# EUROPÄISCHE PATENTSCHRIFT

(11) **EP 0 785 765 B1**
(45) Veröffentlichungstag und Bekanntmachung des Hinweises auf die Patenterteilung: **03.04.2002**
(21) Anmeldenummer: 95934130.6
(22) Anmeldetag: 02.10.1995
(51) Int. Cl.: A61K 7/09

(54) **MITTEL UND VERFAHREN ZUR DAUERHAFTEN VERFORMUNG VON KERATINFASERN**
AGENT AND PROCESS FOR PERMANENTLY SHAPING KERATIN FIBRES
PROCEDE ET AGENTS PERMETTANT DE PERMANENTER DES FIBRES KERATINIQUES

(30) Priorität: 10.10.1994 DE 4436065
(43) Veröffentlichungstag der Anmeldung: 30.07.1997
(73) Patentinhaber: Henkel Kommanditgesellschaft auf Aktien, 40191 Düsseldorf (DE)
(72) Erfinder: OBERKOBUSCH, Doris, D-40591 Düsseldorf (DE); CORTEKAR, Hans-Wolfgang, D-42855 Remscheid (DE); HÖFFKES, Horst, D-40595 Düsseldorf (DE)
(86) Internationale Anmeldenummer: EP9503914
(87) Internationale Veröffentlichungsnummer: WO9610986

(56) Entgegenhaltungen:
- EP-A- 0 091 740
- EP-A- 0 237 870
- EP-A- 0 298 684
- EP-A- 0 363 057
- WO-A-89/05627
- WO-A-92/17155
- DE-A- 2 941 588
- DE-A- 3 138 142
- DE-A- 4 304 828
- DE-A- 4 306 915
- FR-A- 63 804
- FR-A- 64 864
- FR-A- 2 096 687
- FR-A- 2 227 855
- US-A- 2 990 336
- CHEMICAL ABSTRACTS, vol. 91, no. 26, 24. Dezember 1979, Columbus, Ohio, US; abstract no. 216672x, Spalte R ; & JP,A,7 986 635 (AJINOMOTO CO.,INC) 10. Juli 1979
- CHEMICAL ABSTRACTS, vol. 84, no. 22, 31. Mai 1976, Columbus, Ohio, US; abstract no. 155509f, Seite 346 ;Spalte L ; & JP,A,7 615 639 (AJINOMOTO CO.,INC) 7. Februar 1976
- PATENT ABSTRACTS OF JAPAN vol. 5, no. 94 (C-59) (766) 19. Juni 1981 & JP,A,56 039 007 (HOOYUU K.K.) 14. April 1981

## Beschreibung

Die Erfindung betrifft Mittel zur Durchführung der reduzierenden Stufe zur dauerhaften Verformung von Keratinfasern, insbesondere von menschlichen Haaren, sowie die Verwendung dieser Mittel.

Die dauerhafte Verformung von Keratinfasern wird üblicherweise so durchgeführt, daß man die Faser mechanisch verformt und die Verformung durch geeignete Hilfmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung des Keratinreduktionsmittels ist üblicherweise alkalisch eingestellt, damit die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert.

Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Wenngleich dieses als Dauerwelle bezeichnete Verfahren heute in großem Umfang angewendet wird, werden für die reduzierende Stufe nach wie vor Mittel eingesetzt, die hinsichtlich einer Reihe von Punkten nicht als optimal angesehen werden können.

Als Reduktionsmittel werden heute fast ausschließlich Thioglycolsäure oder deren Salze oder Ester verwendet. Die entsprechenden, alkalisch, d. h. bei einem pH-Wert von ca. 8,5 - 9,5, eingestellten Zubereitungen zeigen zwar die gewünschte Reduktionsleistung, können jedoch bei strapaziertem, insbesondere bei oxidativ vorbehandeltem, Haar Schädigungen bis hin zum Haarbruch hervorrufen. Auch können in einzelnen Fällen Probleme im Kopfhautbereich auftreten.

Es gab daher eine Reihe von Versuchen, verbesserte Reduktionsmittel oder Zubereitungen zu entwickeln. Diese Versuche führten jedoch nicht zu befriedigenden Ergebnissen. So verringert eine "saure Einstellung" der Zubereitungen, worunter üblicherweise pH-Werte von 7 - 8 verstanden werden, zwar die Schäden an Haar und Kopfhaut, jedoch ist die Reduktionswirkung am Haar bei üblichen Konzentrationen der Reduktionsmittel unzureichend, so daß die Wellungen zu weich sind und die Dauerwelle nur wenig haltbar ist. Die notwendige Erhöhung dieser Konzentrationen ist aber in vielen Fällen nicht möglich, da nicht zuletzt gesetzliche Auflagen entgegenstehen. Weitere Informationen zu dieser Problematik sind beispielsweise dem "Handbuch der Kosmetik und Riechstoffe" von H. Janistyn, Dr. Alfred Hüthig Verlag Heidelberg, 2.Auflage, 1973, in Band III auf den Seiten 353 und folgenden zu entnehmen.

Aus der deutschen Offenlegungsschrift 36 08 151 ist bekannt, die Wirkung von Haarverformungsmitteln durch Zugabe von 2 bis 25 Gew.-% Dipropylenglykolmonomethylether zu erhöhen.

Aus der Europäischen Patentschrift 363 057 ist bekannt, die Wirkung von Reduktionsmitteln in Dauerwellmitteln in einem pH-Bereich von 3 bis 7 durch Zugabe von 1,3-Alkyldiolen zu steigern.

Aus der japanischen Schrift JP-A-7986635 zitiert in den Chemical Abstracts, Vol. 91 No. 26 vom 24.12.1979, Abstract No. 216672X sowie der japanischen Schrift JP-A-7615639, zitiert in den Chemical Abstracts, Vol. 84, No. 22 vom 31.05.1976, Abstract No. 155509f, der deutschen Offenlegungsschrift DE 3138142 A1 und der WO92/17155 sind Dauerwellmittel enthaltend verschiedene Aminosäuren bekannt.

Aus den französischen Schriften FR 2096687A, FR 0063804A sowie FR 0064864, der deutschen Offenlegung DE 2941588 A1 und der Patentschrift US 2990336, sind Dauerwellmittel enthaltend heterocyclische Verbindungen bekannt.

In den deutschen Offenlegungen DE 4304828 A1 und DE 4306915A1 werden Wellmittel enthaltend Glycin und 1,3-Butandiol offenbart.

Schließlich ist aus WO 89/05627 bekannt, die Verformungswirkung von cysteinhaltigen Haarverformungsmitteln durch Zugabe von Alkandiolen zu erhöhen.

So lassen sich zwar relativ schonende Zubereitungen mit erhöhter Wellaktivität formulieren, jedoch kann die Reduktionswirkung noch immer nicht vollständig befriedigen.

Es wurde nun überraschenderweise gefunden, daß eine wesentliche Verbesserung der Reduktionswirkung praktisch ohne unerwünschte Nebeneffekte an Haar und Kopfhaut durch Zugabe bestimmter Verbindungen zu den reduzierenden Lösungen erreicht werden kann.

Gegenstand der Erfindung ist daher ein Mittel zur Durchführung der reduzierenden Stufe eines Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, dadurch gekennzeichnet, daß die keratinreduzierende Substanz ausgewählt ist aus Thioglykolsäure und Thiomilchsäure sowie deren Alkali- und Ammoniumsalzen und das Mittel mindestens eine, die Wirkung der keratinreduzierenden Substanz verstärkende Verbindung enthält, die ausgewählt ist aus -heterocyclischen Verbindungen (A) aus der Gruppe, die von Imidazol, Mono- und Dialkylimidazolen, Biotin, Hydantoin und Benzimidazol gebildet wird, und Aminosäuren (B), ausgewählt aus Arginin, dessen Salzen und argininreichen Oligopeptiden, jeweils in Kombination mit Diolen.

Im weiteren werden folgende Bezeichnungen verwendet:
- "Wellmittel" für die wäßrige Zubereitungen der keratinreduzierenden Substanz und somit die erfindungsgemäßen Mittel,
- "Zwischenspülung" für die erste Spülung und
- "Fixiermittel" für die wäßrige Zubereitung des Oxidationsmittels.

Weiterhin werden die Einzelheiten der erfindungsgemäßen Lehre anhand von Dauerwellmitteln geschildert. Die entsprechenden Mitteln eignen sich aber in gleichem Maße und mit den gleichen Vorteilen zum Glätten von natürlich gekräuselten oder chemisch gewellten Haaren.

Die erfindungsgemäßen Wellmittel enthalten zwingend die als keratinreduzierende Substanzen bekannten Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Alkalisalze der schwefligen Säure. Bevorzugt geeignet sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 Mol/kg bei einem pH-Wert von 5 bis 12, insbesondere von 7 bis 8,5, eingesetzt.

Als heterocyclische Verbindungen (A) kann Imidazol, eingesetzt werden. Weiterhin eignen sich Derivate dieser Verbindungen wie beispielsweise die C₁₋₄-Alkyl-Derivate, C₁₋₄-Hydroxyalkyl-Derivate und C₁₋₄-Aminoalkyl-Derivate. Bevorzugte Substituenten, die sowohl an Kohlenstoffatomen als auch an Stickstoffatomen der heterocyclischen Ringsysteme positioniert sein können, sind Methyl-, Ethyl-, β-Hydroxyethyl- und β-Aminoethyl-Gruppen. Bevorzugt enthalten diese Derivate 1 oder 2 dieser Substituenten.

Erfindungsgemäß bevorzugte Derivate heterocyclischer Verbindungen sind beispielsweise 1-Methylimidazol, 2-Methylimidazol, 4(5)-Methylimidazol, 1.2-Dimethvlimidazol, 2-Ethylimidazol, 2-Isopropylimidazol, weiterhin erfindungsgemäß bevorzugte Imidazolderivate sind Biotin, Hydantoin und Benzimidazol.

Unter diesen Verbindungen (A) sind die Mono- und Dialkylimidazole, Biotin, Hydantoin, sowie insbesondere das Imidazol selbst besonders bevorzugt.

Diese heterocyclischen Verbindungen (A) sind in den erfindungsgemäßen Wellmitteln in Mengen von 0,5 bis 10 Gewichts-%, bezogen auf das gesamte Wellmittel, enthalten. Mengen von 2 bis 6 Gew.-% haben sich als besonders geeignet erwiesen.

Aus der Gruppe der Aminosäuren (B) hat sich insbesondere Arginin, als erfindungsgemäß geeignet erwiesen. Die Aminosäure kann sowohl als freie Aminosäure, als auch als Salz z. B. als Hydrochlorid eingesetzt werden. Weiterhin haben sich auch Oligopeptide aus durchschnittlich 2-3 Aminosäuren, die einen hohen Anteil (>50%, insbesondere > 70%) an der genannten Aminosäure hat, als erfindungsgemäß einsetzbar erwiesen.

Erfindungsgemäß besonders bevorzugt sind Arginin sowie dessen Salze und argininreiche Oligopeptide.

Diese Aminosäuren bzw. Derivate (B) sind in den erfindungsgemäßen Wellmitteln in Mengen von 0,5 bis 10 Gewichts-%, bezogen auf das gesamte Wellmittel, enthalten. Mengen von 2 bis 6 Gew.-% haben sich als besonders geeignet erwiesen.

Als eine dritte zwingende Komponente haben sich als vorteilhaft Diole erwiesen.

Geeignete Diole sind beispielsweise 2-Ethyl-1,3-hexandiol, 1,3-Butandiol, 1,4-Butandiol, 1,2-Propandiol, 1,3-Propandiol, Neopentylglykol und Ethylenglykol. 1,3-Diole, insbesondere 2-Ethyl-1,3-hexandiol und 1,3-Butandiol, haben sich als besonders gut geeignet erwiesen.

Diese Diole sind in den erfindungsgemäßen Mitteln bevorzugt in Mengen von 2 - 10 enthalten.

Weiterhin können die erfindungsgemäßen Wellmittel alle für Wellmittel bekannten Inhaltsstoffe enthalten, z.B.:
- anionische Tenside wie beispielsweise Seifen, Alkylsulfate und Alkylpolyglykolethersulfate, Salze von Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist, Acylsarcoside, Acyltauride, Acylisethionate, Sulfobernsteinsäuremono- und dialkylester, lineare Alkansulfonate, lineare Alpha-Olefinsulfonate, alpha-Sulfofettsäuremethylester und Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.
- zwitterionische Tenside wie beispielsweise Betaine und 2-Alkyl-3-carboxymethyl-3-hydroxyethyl-imidazoline.
- ampholytische Tenside, wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren.
- nichtionische Tenside wie beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin, C₈-C₂₂-Alkylmono- und -oligoglycoside und deren ethoxylierte Analoga sowie Anlagerungsprodukte von 5 bis 60 Mol Ethylenoxid an Rizinusöl und gehärtetes Rizinusöl.
- kationische Tenside, wie quartäre Ammoniumverbindungen mit 1-2 C₁₂₋ ₁₈-Alkylketten und 2 - 3 C₁₋₄-Alkylketten, Pyridinium- und Imidazoliniumsalze.
- Proteinhydrolysate wie beispielsweise Kollagen-Hydrolysate, Elastin-Hydrolysate, Keratin-Hydrolysate, Hydrolysate von Weizenproteinen, Milchproteinen, Eiweißproteinen, Seidenproteinen, Mandelproteinen, Sojaeiweiß sowie Proteinen aus Tierhäuten, Kollagenhydrolysat-Kondensate mit organischen Säuren, wie beispielsweise Ölsäure, Myristinsäure, Undecylensäure, Kokosfettsäure und Abietinsäure, und deren Salze, Elastinhydrolysat-Kondensate mit Fettsäuren und kationische Kollagenhydrolysate.
- Verdickungsmittel wie Agar-Agar, Guar-Gum, Alginate, Xanthan-Gum und Cellulose-Derivate wie Hydroxyethyl- und Methylhydroxypropyl-Cellulose,
- Strukturanten wie Glucose und Maleinsäure,
- Kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- Anionische, zwitterionische, amphotere und nichtionische Polymere wie beispielsweise Vinylacetat/Crotonsäure-Copolymere, Vinylpyrrolidon/ Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyl-trimethylammoniumchlorid/Acrylat-Copolymere, Octylacrylamid/Methyl-methacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/ Vinylcaprolactam-Terpolymere sowie gegebenfalls derivatisierte Celluloseether und Silikone.
- Lösungsvermittler, wie Ethanol, Isopropanol, Glycerin und Diethylenglykol,
- Substanzen zur Einstellung des pH-Wertes wie Natronlauge, Ammoniak, Ammoniumcarbonat, Ammoniumcarbamat und Citronensäure/Natriumcitrat-Puffer,
- Wirkstoffe wie Panthenol, Allantoin, Pyrrolidoncarbonsäuren, Pflanzenextrakte und Vitamine,
- Lichtschutzmittel,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Farbstoffe,
- Parfümöle, Dimethylisosorbid und Cyclodextrine,
- Perlglanzmittel wie Ethylenglykolmono- und -distearat sowie Fettsäurealkanolamide
- Treibmittel wie Propan-Butan-Gemische, N₂O, Dimethylether und Luft

Die Einwirkungszeit der Wellmittel auf dem Haar beträgt in der Regel 15 bis 40 Minuten, wobei die Beschaffenheit des Haares, die bereits durchgeführten chemischen Haarbehandlungen, der gewünschte Verformungsgrad, die Größe der verwendeten mechanischen Verformungshilfe (Haarwickler) und die Art des Keratinreduktionsmittels weitere Einflußgrößen sind.

Die erfindungsgemäßen Wellmittel können als gebrauchsfertige Mischungen formuliert werden, die vom Friseur oder Endverbraucher direkt angewendet werden können. Es hat sich in manchen Fällen aber als vorteilhaft oder notwendig erwiesen, wenn die Mittel als sogenannte 2-Komponenten-Mischungen formuliert werden, die erst vom Anwender zum gebrauchsfertigen Wellmittel vermischt werden. In diesem Fall enthält eine Formulierung das Reduktionsmittel in einem geeigneten Träger, z.B. Wasser oder einer Emulsion.

Die erfindungsgemäßen Wellmittel können mit beliebigen Fixiermitteln kombiniert werden.

Zwingender Bestandteil der Fixiermittel sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger H₂O₂-Zubereitungen, die üblicherweise etwa 0,5 bis 3,0 Gew.-% H₂O₂ enthalten, liegt bevorzugt bei 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (Peroxidasen), die keine oder nur geringe Mengen an Oxidationsmitteln, insbesondere H₂O₂, enthalten.

Die erfindungsgemäßen Fixiermittel können als Feststoffe formuliert werden. Sie enthalten das Oxidationsmittel dann in Form eines Festkörpers, z.B. Kalium- oder Natriumbromat. Erst kurz vor der Anwendung werden diese Mittel dann mit Wasser versetzt. Es kann ebenfalls bevorzugt sein, das Oxidationsmittel als 2-Komponenten-System zu formulieren. Die beiden Komponenten, von denen die eine bevorzugt eine Wasserstoffperoxidlösung oder eine wäßrige Lösung eines anderen Oxidationsmittels ist und die andere die übrigen Bestandteile enthält, werden ebenfalls erst kurz vor der Anwendung vermischt.

Beispiele für die in den erfindungsgemäßen Haarbehandlungsmitteln verwendbaren kationischen Tenside sind insbesondere quartäre Ammoniumverbindungen, Alkylamidoamine sowie die sogenannten Esterquats wie die unter dem Warenzeichen Stepantex^{R} vertriebenen Dialkylammoniummethosulfate und Methyl-hydroxyalkyl-dialkoyloxyalkyl-ammoniummethosulfate.

Die erfindungsgemäßen Zwischenspülungen werden üblicherweise mit Wasser durchgeführt, dem anorganische Salze zugefügt werden können.

Sowohl Wellmittel als auch Fixiermittel können als Creme, Gel oder Flüssigkeit formuliert sein. Weiterhin ist es möglich, die Mittel in Form von Schaumaerosolen zu konfektionieren, die mit einem verflüssigten Gas wie z. B. Propan-Butan-Gemischen, Stickstoff, CO₂, Luft, N₂O, Dimethylether, Fluorchlorkohlenwasserstofftreibmitteln oder Gemischen davon in Aerosolbehältern mit Schaumventil abgefüllt werden.

Die erfindungsgemäßen Wellmittel können mit allen, dem Fachmann bekannten, üblichen Vorbehandlungsmitteln, Zwischenspülungen und/oder Nachbehandlungsmitteln (zur Verbesserung von Avivage und Haltbarkeit der Frisur) kombiniert werden.

Gegenstand der Erfindung ist auch die Verwendung eines Mittels nach einem der Ansprüche 1 bis 8 zur Durchführung der reduzierenden Stufe eines Verfahrens zur dauerhatten Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült.

### Vorschrift zur Bestimmung des Wellwertes nach Kirby

Zur Messung wurde eine 0,5 g schwere und 25 cm lange, glatte, an beiden Enden gleich dick abgebundene Haarsträhne mit der Wellflüssigkeit befeuchtet. Diese wurde eng, aber ohne Spannung, um die Stifte des Wellbrettes gelegt, nochmals mit Wellflüssigkeit getränkt und 30 Min. bei 37 °C gewellt. Die Strähne wurde auf dem Brett gespült, 10 Min. in eine Fixierlösung von Zimmertemperatur getaucht, gespült, dann erst vorsichtig vom Wellbrett gelöst und 5 Min. in ein Wasserbad von 30 °C gelegt. Die lineare Entfernung der 1. und der 6. Welle (5 Wellen !) = Bₒ = Wellwert wurde gemessen. Weitere Einzelheiten zum Verfahren sind der Veröffentlichung von D.H. Kirby KPDR Heft 1/2, Seiten 3-6 (1958) zu entnehmen.

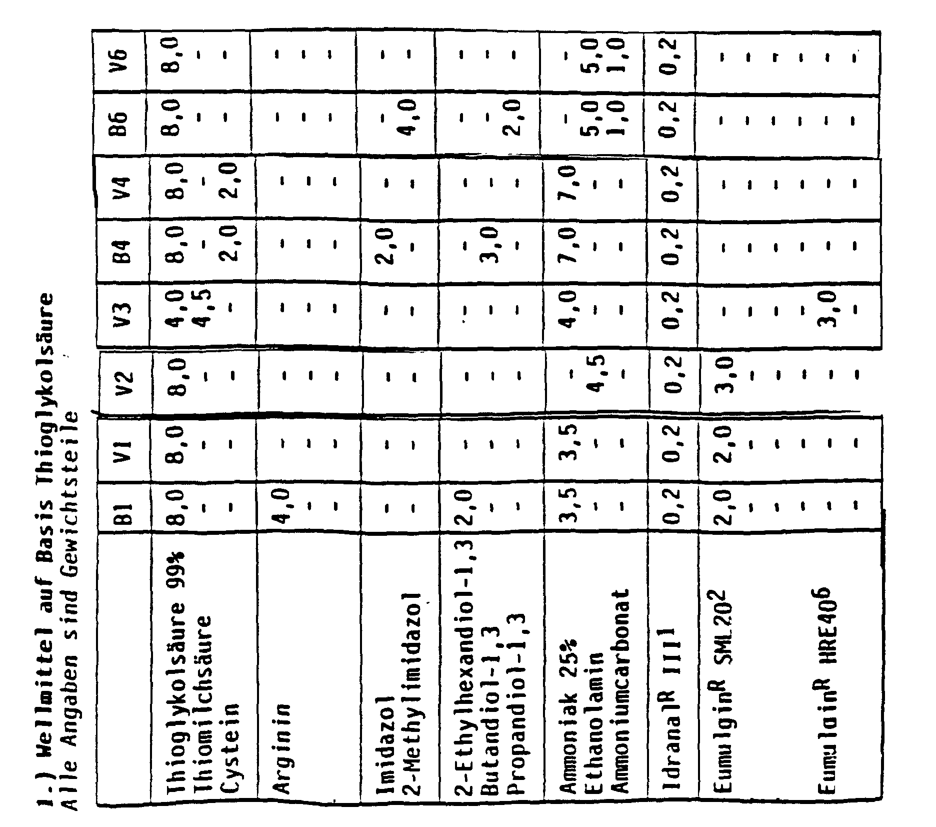

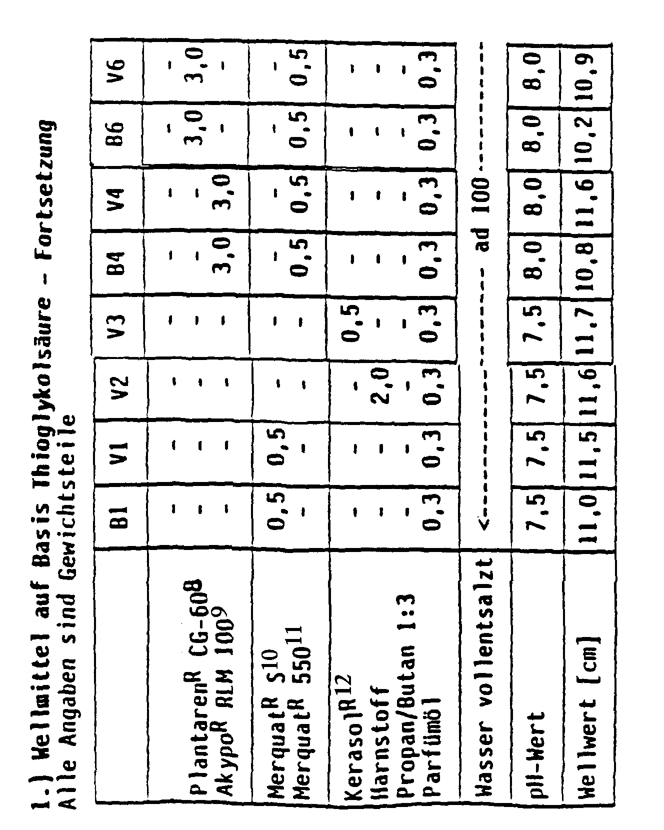

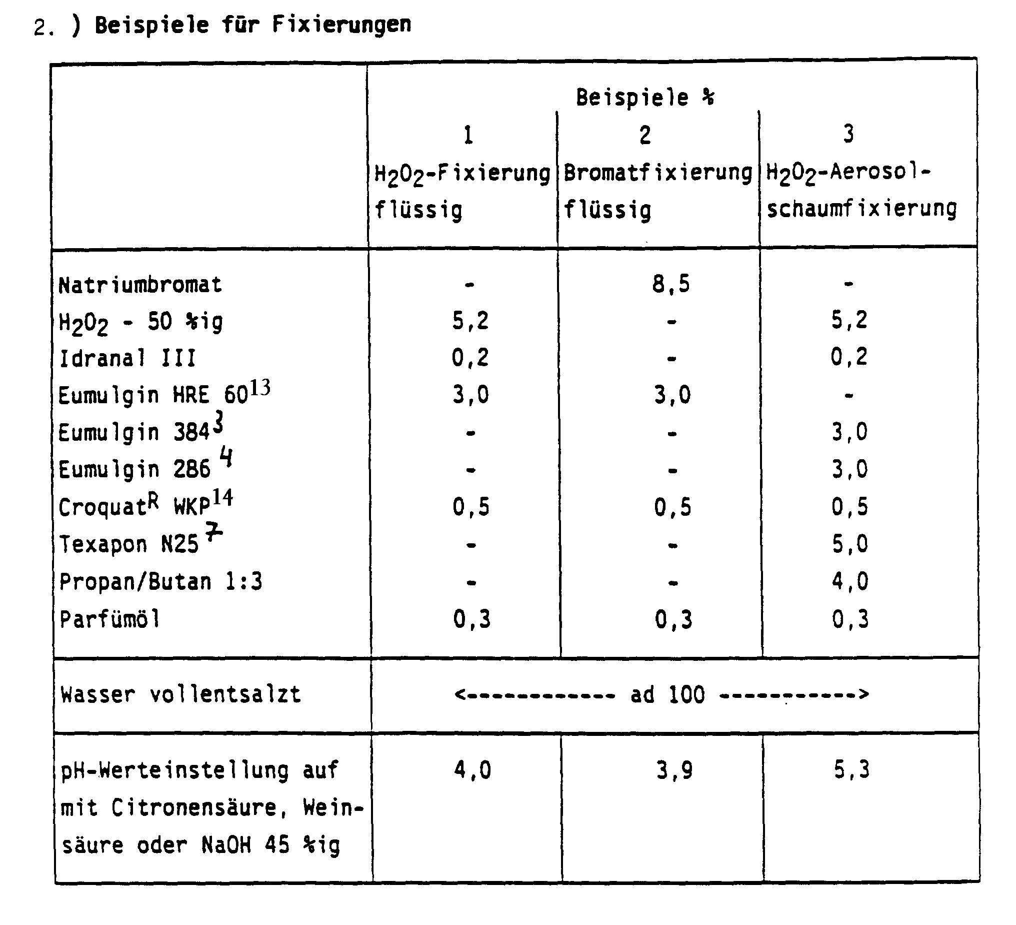

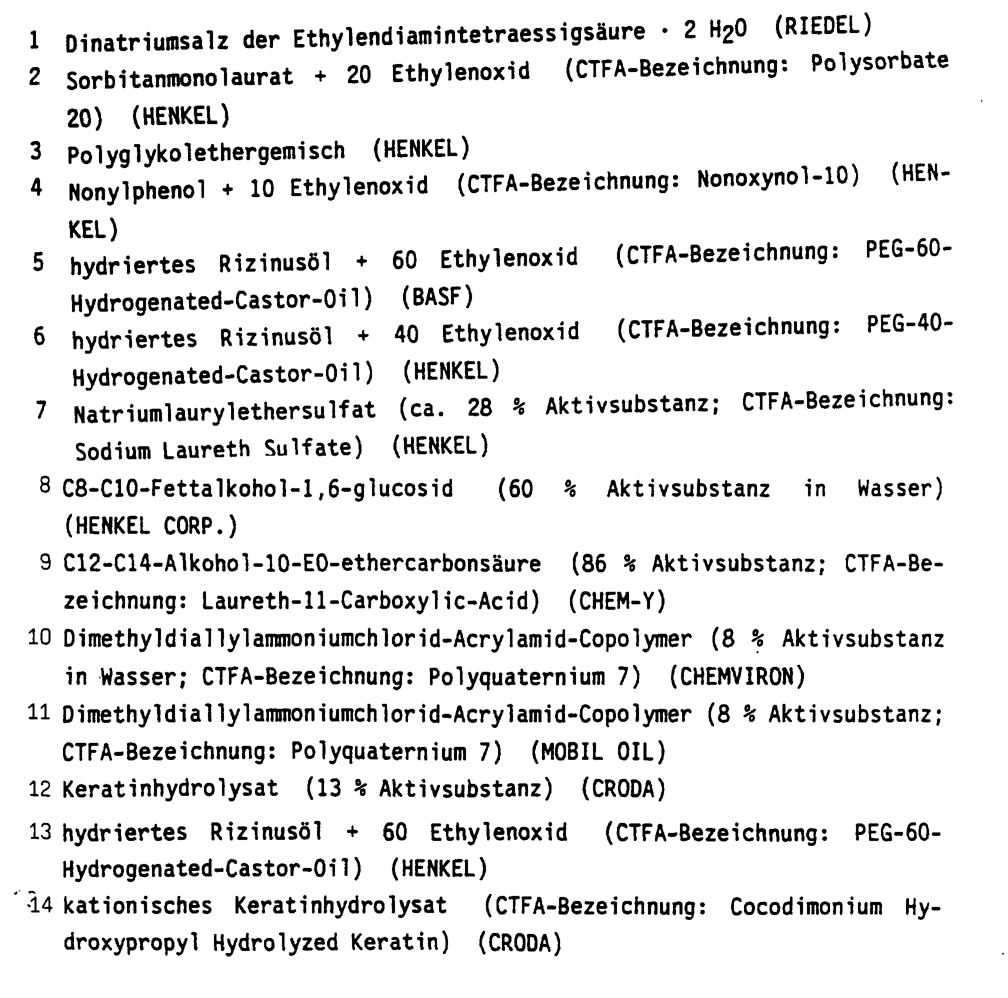

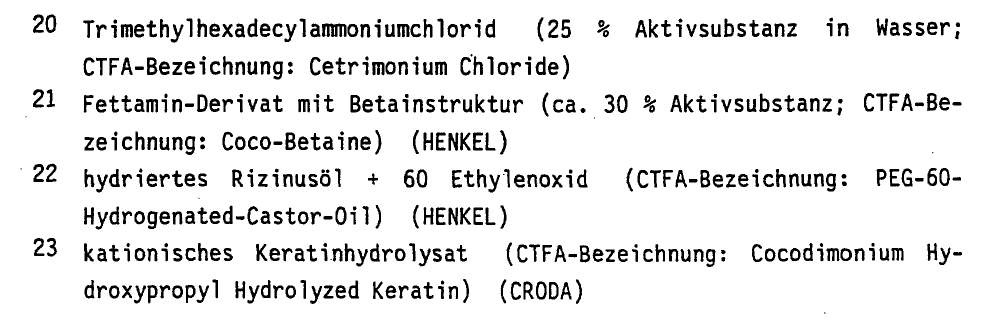

## Patentansprüche

1. Mittel zur Durchführung der reduzierenden Stufe eines Verfahren zur dauerhaften Verformung von Keratinfasem, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, **dadurch gekennzeichnet, daß** die keratinreduzierende Substanz ausgewählt ist aus Thioglykolsäure und Thiomilchsäure sowie deren Alkali- und Ammoniumsalzen und das Mittel mindestens eine die Wirkung der keratinreduzierenden Substanz verstärkende Verbindung enthält, die ausgewählt ist aus
- heterocyclischen Verbindungen (A) aus der Gruppe, die von Imidazol, Mono- und Dialkylimidazolen, Biotin, Hydantoin und Benzimidazol gebildet wird, und
- Aminosäuren (B), ausgewählt aus Arginin, dessen Salzen und argininreichen Oligopeptiden, jeweils in Kombination mit Diolen.

2. Mittel nach Anspruch 1, **dadurch gekennzeichnet, daß** die heterocyclische Verbindung (A) ausgewählt ist aus Imidazol sowie Mono- und Di-alkylimidazolen.

3. Mittel nach einem der Ansprüche 1 oder 2, **dadurch gekennzeichnet, daß** die heterocyclische Verbindung (A) Imidazol ist.

4. Mittel nach einem der Ansprüche 1 bis 3, **dadurch gekennzeichnet, daß** die heterocyclische Verbindung in Mengen von 0,5 - 10 Gew.-%, insbesondere in Mengen von 2 - 6 Gew.-%, bezogen auf das gesamte Mittel, enthalten ist.

5. Mittel nach einem der Ansprüche 1 bis 4, **dadurch gekennzeichnet, daß** die Aminosäure oder das Aminosäurederivat (B) in Mengen von 0,5 - 10 Gew.-%, insbesondere in Mengen von 2 - 6 Gew.-%, bezogen auf das gesamte Mittel, enthalten ist.

6. Mittel nach einem der Ansprüche 1 bis 5, **dadurch gekennzeichnet, daß** das Diol ausgewählt ist aus 2-Ethyl-1,3-hexandiol, 1,3-Butandiol und 1,2-Propylenglykol.

7. Mittel nach einem der Ansprüche 1 bis 6, **dadurch gekennzeichnet, daß** es einen pH-Wert von 5 - 12, insbesondere von 7 - 8,5, aufweist.

8. Mittel nach einem der Ansprüche 1 bis 7, **dadurch gekennzeichnet, daß** es als Schaumaerosol formuliert ist.

9. Verwendung eines Mittels nach einem der Ansprüche 1 bis 8 zur Durchführung der reduzierenden Stufe eines Verfahrens zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült.

## Claims

1. Composition for carrying out the reducing step of a process for permanently deforming keratin fibers in which the fibers are treated with an aqueous preparation of a keratin-reducing substance before and/or after a mechanical deforming step, rinsed after a contact time with a first rinse, then fixed with an aqueous preparation of an oxidizing agent and rinsed again after another contact time, **characterized in that** the keratin-reducing substance is selected from thioglycolic acid and thiolactic acid and alkali metal and ammonium salts thereof and **in that** the composition contains at least one compound which strengthens the effect of the keratin-reducing substance and which is selected from
- heterocyclic compounds (A) from the group consisting of imidazole, mono- and dialkyl imidazoles, biotin, hydantoin and benzimidazole and
- amino acids (B) selected from arginine, its salts and arginine-rich oligopeptides - all in combination with diols.

2. Composition as claimed in claim 1, **characterized in that** the heterocyclic compound (A) is selected from imidazole and mono- and dialkyl imidazoles.

3. Composition as claimed in claim 1 or 2, **characterized in that** the heterocyclic compound (A) is imidazole.

4. Composition as claimed in any of claims 1 to 3, **characterized in that** the heterocyclic compound is present in quantities of 0.5 to 10% by weight and more particularly in quantities of 2 to 6% by weight, based on the composition as a whole.

5. Composition as claimed in any of claims 1 to 4, **characterized in that** the amino acid or the amino acid derivative (B) is present in quantities of 0.5 to 10% by weight and more particularly in quantities of 2 to 6% by weight, based on the composition as a whole.

6. Composition as claimed in any of claims 1 to 5, **characterized in that** the diol is selected from 2-ethylhexane-1,3-diol, butane-1,2-diol and 1,2-propylene glycol.

7. Composition as claimed in any of claims 1 to 6, **characterized in that** it has a pH of 5 to 12 and more particularly 7 to 8.5.

8. Composition as claimed in any of claims 1 to 7, **characterized in that** it is formulated as a foam aerosol.

9. The use of the composition claimed in any of claims 1 to 8 for carrying out the reducing step of a process for permanently deforming keratin fibers in which the fibers are treated with an aqueous preparation of a keratin-reducing substance before and/or after a mechanical deforming step, rinsed after a contact time with a first rinse, then fixed with an aqueous preparation of an oxidizing agent and rinsed again after another contact time.

## Revendications

1. Agent d'exécution de l'étape de réduction d'un procédé de mise en forme durable des fibres de kératine, dans lequel on traite les fibres avant et/ou après une mise en forme mécanique, avec une préparation aqueuse d'une substance réductrice de kératine, on rince après un temps d'action avec un premier liquide de rinçage, puis on fixe avec une préparation aqueuse d'un agent d'oxydation et également on rince après un temps d'action,
**caractérisé en ce que**
la substance réductrice de kératine est choisie parmi l'acide thioglycolique et l'acide thiolactique ainsi que parmi leurs sels de métal alcalin et d'ammonium, et l'agent contient au moins un composé qui renforce l'action de la substance réductrice de kératine, composé choisi parmi,
- les composés hétérocycliques (A) choisis dans le groupe formé par l'imidazole, les mono- et dialkylimidazoles, la biotine, l'hydantoine, et le benzimidazole, et
- les aminoacides (B) choisis parmi l'arginine, ses sels et les oligopeptides riches en arginine à chaque fois en combinaison avec des diols.

2. Agent selon la revendication 1,
**caractérisé en ce que**
le composé hétérocyclique (A) est choisi parmi l'imidazole ainsi que parmi les mono- et les di-alkylimidazoles.

3. Agent selon l'une quelconque des revendications 1 ou 2,
**caractérisé en ce que**
le composé hétérocyclique (A) est l'imidazole.

4. Agent selon l'une quelconque des revendications 1 à 3,
**caractérisé en ce que**
le composé hétérocyclique est contenu en quantités allant de 0,5 à 10 % en poids, en particulier en quantités de 2 à 6 % en poids, rapporté à la totalité de l'agent.

5. Agent selon l'une quelconque des revendications 1 à 4,
**caractérisé en ce que**
l'aminoacide ou le dérivé d'aminoacide (B) est contenu en quantités allant de 0,5 à 10 % en poids, en particulier en quantités de 2 à 6 % en poids rapporté à la totalité de l'agent.

6. Agent selon l'une quelconque des revendications 1 à 5,
**caractérisé en ce que**
le diol est choisi parmi le 2-éthyl-1,3-hexanediol, le 1,3-butanediol et le 1,2-propylèneglycol.

7. Agent selon l'une quelconque des revendications 1 à 6,
**caractérisé en ce qu'**
il manifeste une valeur de pH de 5 à 12, en particulier de 7 à 8,5.

8. Agent selon l'une quelconque des revendications 1 à 7,
**caractérisé en ce qu'**
il est formulé sous forme d'aérosol en mousse.

9. Utilisation d'un agent selon l'une quelconque des revendications 1 à 8, destiné à l'exécution de l'étape réductrice d'un procédé en vue de la mise en forme de fibres de kératine, procédé dans lequel on traite les fibres avant et/ou après une mise en forme mécanique, avec une préparation aqueuse d'une substance réductrice de kératine, on rince avec un temps d'action avec un premier liquide de rinçage puis on fixe avec une préparation aqueuse d'un agent d'oxydation et on rince également après un temps d'action.
